# NEW EUROPEAN PATENT SPECIFICATION

(11) **EP 0 063 891 B2**
(45) Date of publication and mention of the opposition decision: **27.03.1996**
(45) Mention of the grant of the patent: 28.02.1990
(21) Application number: 82301882.5
(22) Date of filing: 08.04.1982
(51) Int. Cl.: A61C 5/06

(54) **Ejector holder for capsule-like cartridge**
Presse für eine kapselähnliche Patrone
Support-éjecteur pour cartouche analogue à une capsule

(30) Priority: 09.04.1981 US 252558; 29.01.1982 US 344254; 29.01.1982 US 344255
(43) Date of publication of application: 03.11.1982
(60) Divisional application: 84113283.0
(73) Proprietor: DENTSPLY INTERNATIONAL, INC., York Pennsylvania 17405 (US)
(72) Inventor: Dougherty, Emery W., York Pennsylvania 17402 (US); Welsh, Richard E., Milford Delaware 19963 (US)
(74) Representative: Wächtershäuser, Günter, Prof. Dr.

(56) References cited:
- BR-A- 5 701 465
- FR-A- 2 078 627
- GB-A- 298 292
- US-A- 1 926 496
- US-A- 2 505 028
- US-A- 3 076 455
- US-A- 3 184 120
- US-A- 3 220 412
- US-A- 3 300 100
- US-A- 3 464 412
- US-A- 3 534 735
- US-A- 3 581 399
- US-A- 3 815 878
- US-A- 3 900 954
- US-A- 3 907 106
- US-A- 4 198 756
- US-B- 4 330 280

## Description

This invention is directed to a manually operable ejector holder in combination with a cartridge for use in connection with the ejection of viscous dental materials.

In recent years it has become popular to package various types of material, especially medicinal or quasi-medicinal types in sealed cartridges, insertable in a suitable type of holder and/or ejector device, for purposes of preserving purity of the medicament and the like, insuring a patient of accurately measured quantities, as well as minimizing effort now required in introducing bulk amounts of material into syringes and ejecting measured quantities thereof, for example. Various previous efforts in this direction are illustrated and described in various prior U.S. patents, particularly U.S. Patent No. 3,581,399 to Dragan, dated June 1, 1971, in which a typical example of loaded cartridge is illustrated in conjunction with one type of holder and discharge device.

Other efforts have been made to produce similar devices, one of these comprising the subject matter of prior U.S. Patent No. 3,900,954, also to Dragan, dated Aug. 26, 1975, and comprising a simpler version than in Dragan's patent No. 3,581,399. Also, another U.S. patent to Dragan, No. 4,198,756, dated April 22, 1980 shows an ejector design for the aforementioned cartridges.

It has been found in the operation of the Dragan devices particularly relative to the curved discharge end of the capsule or cartridges that there have been occasions when the leading end of the ejecting plunger or the piston within the cartridge pushed through the wall adjacent the outer end of the cartridge. Particularly for purposes of obviating this difficulty and also for providing what is believed to be a simple and improved compartment at the forward end of the barrel of the holder, as well as also providing an improved cartridge not subject to the difficulties of Dragan's cartridges, which is free of difficulties similar to those described with respect to the Dragan cartridge, the present invention has been devised and details thereof are set forth hereinbelow. Certain other constructions for cartridges of the type described above are disclosed in U.S. Patent 2,505,028 to Boeger issued April 25, 1950.

MU―57 01 465 discloses a manually operable ejector holder comprising an elongate barrel having a forward end provided with a longitudinal bore a plunger reciprocable therein. The holder further comprises an element movable relative to the barrel for moving the plunger.

U.S. 3 076 455 and U.S. 3 220 455 disclose a manually operable hypodermic syringe for capsuled medicaments. The syringe comprises a holder for securing a capsule, plunger for forcing the composition from the capsule and an element movable relative to the holder for moving the plunger.

The present invention also comprises a simplified improvement over U.S. patent No. 4,295,828 in the name of Helmut Rudler, dated October 20, 1981, and entitled 'Ejector Holder for Syringe-type Cartridge', the invention covered thereby being assigned to the same assignee as the invention of the instant application.

It is among the principal objects of the present invention to provide preferably a one-piece barrel having integral and relatively simple means at the forward end thereof to seat and retain an improved cartridge having an annular flange at the end opposite the discharge end, said cartridge being retained by a simple snap-acting arrangement.

It is another object of the invention to provide at the forward end of said barrel a compartment or bore in which said aforementioned seat for the flange of the cartridge is included, said compartment being formed simply by cutting away part of the wall comprising the forward end of the barrel a limited distance inwardly and axially from the outer end of the barrel, the surface formed by the cutaway arrangement lying within a plane parallel to the axis and radially spaced from the same a short distance, the inner end portion of the cutaway arrangement being wider than the portion extending forwardly therefrom for purposes of receiving the flange of the cartridge in a seat for said flange which is forwardly of the inner end of the compartment and into which the flange is inserted to mount the cartridge in the compartment.

A further object of the invention is to provide in the compartment a semi-cylindrical surface from which opposite, substantially parallel sidewalls extend and the upper edges of said sidewalls extending a very limited distance toward each other and said sidewalls having limited flexibility to provide the snap acting retaining arrangement referred to above.

Still another object of the invention is to provide on the rearward end of the barrel a handle fixed thereto and extending transversely thereto, preferably in opposite direction from the axis of the barrel, a plunger being reciprocably mounted in the barrel for engagement at one end with a combination closure plug and piston in a cartridge, when mounted in the forward end of the barrel, and several embodiments of an actuating lever are provided which respectively are a lever of the second class and are pivotally associated with one end of said handle and extend from the pivot means thereof past the opposite end of the plunger and engaging the same for reciprocation thereof, as aforesaid, when the lever is manually activated toward the handle, spring means being provided on the plunger to retract the same from engagement of the forward end thereof with a cartridge.

Another object closely ancillary to the foregoing object is to provide one embodiment of lever which has affixed pivot axis relative to one end of the handle, whereby the mid-portion of the lever has a sliding engagement with the aforesaid opposite end of the plunger, while another embodiment of lever has a slideable pivot fulcrum relationship with the associated end of the handle and said opposite end of the plunger has a rocking engagement with an intermediate portion of the lever.

Still another object of the invention, also ancillary to the several foregoing objects, and especially the latter embodiment of lever which has a rocking engagement with the opposite end of the plunger, is to provide an engagement between said opposite end of the plunger and intermediate portion of said lever which is in the nature of a partial ball and socket configuration, whereby the lever pivotally moves about the centre of the partial ball-like member, while the slidable pivot fulcrum end of the handle moves relative to the pivot axis thereof.

These objects are achieved by the ejector holder of claim 1. Preferably the undercut groove at the upper ends thereof has opposite wall portions in said compartment extending toward each other a limited distance less than the diameter of the annular cartridge collar to provide a seat for said collar forward of the portion of said laterally recessed portion of said sidewalls of said compartment and from which said collar cannot be removed laterally.

According to one advantageous embodiment the lever is connected to one end of said handle by pivotal means and extending from said pivotal means past said projecting end of said plunger, said lever having an arcuate surface, intermediately of the ends thereof, engaging said projecting end of said plunger.

A cartridge for use in the ejector holder of the present invention is the subject of European Patent Application No. 0145922, which is a divisional application based upon the present application.

The invention will now be described by way of example only with reference to the several figures of the accompanying drawings in which:―
Figure 1 is a side elevation of an ejector holder supporting a capsule-like cartridge in accordance with the principles of the present invention;
Figure 2 is a top plan view of the holder and cartridge shown in Figure 1;
Figure 3 is a fragmentary enlarged bottom plan view of the forward end of the barrel of the ejector holder shown in Figures 1 and 2;
Figure 4 is a fragmentary bottom plan view of the ejector holder similar to Figure 3, but on a smaller scale, and illustrating a cartridge supported in the forward end of the barrel;
Figure 5 is a vertical sectional view of the forward end portion of the barrel of the ejector holder shown in Figure 3, as taken on section station 5―5 thereof;
Figure 6 is a front end view of the forward end of the barrel shown in Figures 1―4;
Figure 7 is a side elevation, partly broken away, of a cartridge similar to that shown in Figures 1, 2 and 4, but on a larger scale, and illustrating a piston inserted in the open end of the cartridge and also showing fragmentary a portion of a plunger rod of the ejector holder adapted to engage said piston;
Figure 8 is a side elevation of another embodiment of ejector in accordance with this invention;
Figure 9 is a top plan view of Figure 8; and
Figure 10 is a vertical section of Figure 9 as taken on section station 10―10 thereof.

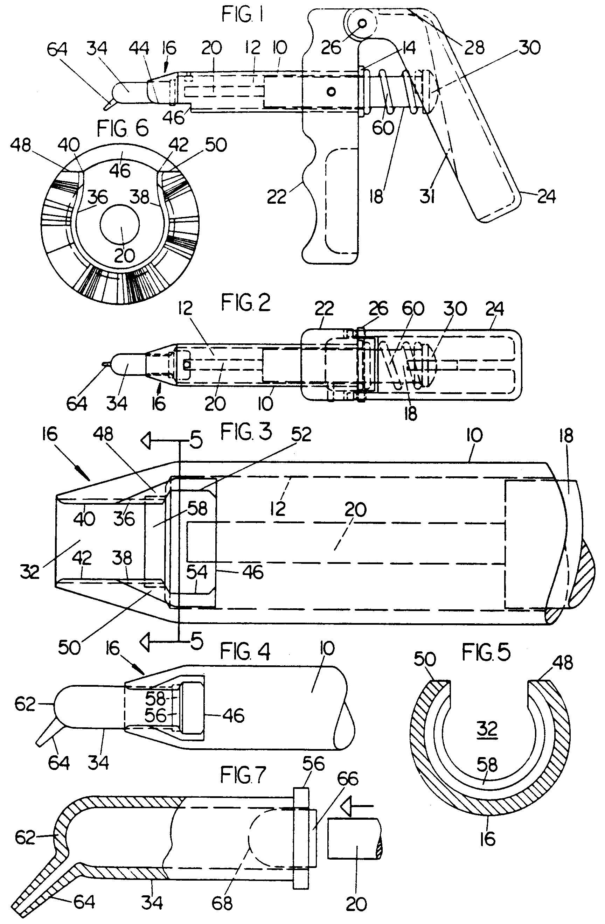

Referring now to Figure 1, there is shown therein an ejector holder which includes the principles of one embodiment of the present invention and comprising a barrel 10 having an interior bore 12 extending from the rearward end 14 of the barrel toward the forward end 16 thereof for purposes of receiving a plunger 18 of the same diameter as that of the interior bore 12 for the major portion of the length of the plunger, the forward end of the plunger having a smaller diameter extension 20.

The rearward end 14 of the barrel 10 extends through and is fixed to a handle member 22 with which the barrel 10 is perpendicular. Pivotally connected to the handle 22 is an operating lever 24, the upper end of which is pivotally connected to the upper end of handle 22 by a pivot pin 26 which is fixed relative to the upper end of handle member 22. The upper end 28 of operating lever 24 is offset laterally to facilitate operation of the lever 24 with respect to the outer end of plunger 18 which terminates in a button 30, which is slidably engaged by a portion of the inner surface 31 of operating lever 24.

From Figs. 1―4, it will be seen that the forward end 16 of the barrel 10 is tapered and is provided with a longitudinally extending opening comprising compartment 32 which extends rearwardly from the terminal end of the forward end 16 toward the interior bore 12. The lower surface of compartment 32, as viewed in Fig. 3, is semi-cylindrical and is complementary to the elongated body of cartridge 34 so as to receive and seat the same, as shown in Figs. 1, 2, and 4. The sidewalls 36 and 38 of compartment 32 extend upwardly from the semi-cylindrical bottom surface shown in Fig. 3 and are parallel to each other for a limited distance and the upper edges 40 and 42 extend toward each other a limited distance. Said uppermost portions of sidewalls 36 and 38 also have limited flexibility, whereby the distance between the upper edges 40 and 42 of said sidewalls preferably is slightly less than the diameter of the cartridge 34, whereby there is a snap-acting retaining function provided by said sidewalls and the upper edges 40 and 42 with respect to the cartridge 34 when the latter is inserted in the compartment 32.

The forward end 16 of the barrel 10 also has a cutaway portion 44 extending longitudinally rearward to form a shoulder 46, which determines the inner end of the cutaway portion. Due to the fact that the forward end 16 is tapered and the barrel 10 otherwise is circular, said cutaway arrangement provides flat surfaces 48 and 50. Also, as best shown in Fig. 3, the sidewalls of the compartment 32, at the inner ends thereof, have lateral recesses 52 and 54 which are spaced apart a greater distance than the diameter of the annular exterior flange 56 in order to permit the insertion of the flange into compartment 32 which, following radial insertion movement thereof into the compartment, the cartridge may be moved axially forward for disposition of the flange 56 in an undercut seat 58, which is clearly shown in Figs. 3―5. Said seat, in conjunction with the portion of the compartment 32 extending forwardly therefrom, provides a firm means for supporting a cartridge 34, which is retained seated in said compartment, especially by means of the snap-fitting arrangement provided by the upper edges 40 and 42 of the sidewalls 36 and 38, as described hereinabove.

Without restriction thereto, the preferred material from which the barrel 10, handle member 22 and operating lever 24 are formed, is a suitable rigid plastic material in order that these elements may be formed readily and accurately by molding from raw plastic material; obviously, the coiled spring 60 is formed from spring wire for purposes of retracting the plunger 18 when the operating lever 24 is released, following an ejection of material from the cartridge 34.

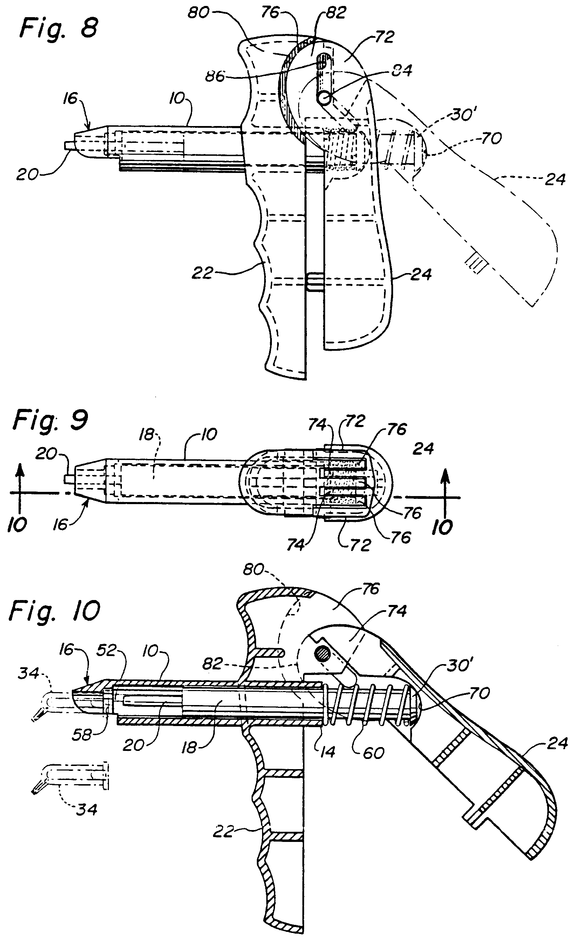

The cartridge 34 also may be formed by molding from appropriate, preferably rigid, synthetic resin or plastic material by means of a suitable mold. The intermediate body portion of the capsule 34 is of uniform interior and exterior diameter and extends from the flange 56 adjacent the open end of the cartridge to the opposite closed end 62. The body portion is cylindrical, whereas the closed end 62 is hemispherical but is provided with an angularly extending discharge nipple 64, the opening of which is preferably a very fine dimension of small diameter. To effect ejection of material from the cartridge 34, such as dental filling material, cement, or other viscous dental material and the like, the cartridge 34 includes a piston 66, which is closely complementary in diameter to the interior of the cartridge 34, and the inner end 68 thereof also is hemispherical and complementary to the interior of the closed end 62 of the cartridge. Without restriction thereto, the outer end of the piston may be flat for engagement, for example, with the extension 20, shown fragmentarily in Fig. 7, when the plunger 18 is moved forwardly by actuation of the operating lever 24.

Removal of the capsule 34 from the compartment 32 is accomplished readily by snapping the cartridge outwardly beyond the somewhat flexible upper edges 40 and 42 of the compartment after the contents within the cartridge have been discharged or exhausted, as required.

Another embodiment of ejector from that shown in Figs. 1―7 is illustrated in Figs. 8―10, both embodiments utilizing the cartridge-attaching means shown at the forward or left-hand end of the barrel 10 as illustrated in Figs. 8―10 in the second embodiment and, correspondingly, in Figs. 1―7 of the first embodiment. In Figs. 8―10, it will be seen that the handle 24 is fixedly connected to the rearward end of the barrel 10 in the same manner as the corresponding element in Figs. 1―7. However, the operating lever 24 is connected to the handle member 22 in a different manner from that illustrated in Figs. 1―7, details of which are as follows:

The plunger 18 is reciprocable in the barrel 10 in opposite direction for purposes of moving the extension 20 at the forward end thereof toward the cartridge 34 for purposes of effecting discharge thereof. The rearward or right-hand end of the plunger 18, as viewed in Figs. 8 and 10, is provided with a button 30' which is slightly different from the button 30 shown in Figs. 1―7, in that the same actually constitutes a part of a ball and socket connection, of which the button 30' is a fragmentary ball or at least functions as a fragmentary ball, the perimeter of which has a greater diameter than that of the plunger 18 in order that the compression spring 60 may be disposed with its opposite end respectively between the button 30' and the rearward end 14 of the barrel 10. The other part of the fragmentary ball and socket joint or connection comprises a somewhat hemispherical seat 70 formed in the inner portion of the lever 24 intermediately of the ends thereof, whereby the modified button 30' actually comprises a fulcrum about which the seat 70 rotates within the same plane as the handle 22 and lever 24, the center of rotation being coincident with the axis of the plunger 18.

It has been found that movement of the lever 24 toward and from the handle 22 is facilitated by the more-or-less fragmentary ball and socket joint or connection comprising the button 30' and seat 70 when engaging the handle and lever somewhat in pistol-gripping manner by one hand of an operator, especially when moving the lever 24 from the extended position shown in Fig. 10 to the fully closed position shown in Fig. 8, the initial position also being shown in Fig. 8 in phantom.

In order to permit the operation of the lever 24 in the manner just described, however, the upper ends of both the hand 22 and lever 24 are modified from the arrangement shown in the embodiment of Figs. 1―7, in the following respects:
Especially as shown in Fig. 9, it will be seen that the upper end of lever 24 is provided with a plurality of similar parallel leaves 72 and 74, the leaves 72 being outermost and the leaves 74 being disposed inwardly thereof. Interdigitated with the leaves 72 and 74 of lever 24 are a plurality of three similar leaves 76 on the upper end of handle 72. Further, the exterior side 78 of the upper end of handle 22 respectively is provided with relatively shallow and flat arcuate sockets 80, see Fig. 8, to accommodate the corresponding arcuate configurations 82 of lever 24.

In view of the pivotal movement of lever 24 about the axis of the modified button 30' and the plunger 18, it will be seen that the upper end of lever 24, which actually is a lever of the second class, is provided with a floating fulcrum in the form of a pin 84 which extends transversely through the leaves 76 of handle 22, and each of the leaves 72 and 74 of the lever 24 are provided with slots 86 which are all parallel to each other and commonly receive the pin 84 and thereby provide the aforementioned floating fulcrum for the lever 24 and easier hand operation thereof.

From the foregoing, it will be seen that the several embodiments of ejector holders and the particular type of cartridge to be used therewith are of very simple, but highly effective design, to permit sure and quick mounting of the cartridge within the compartment in the forward end of the barrel of the holder and, with equal facility, removal of the cartridge therefrom is readily achieved. Assembly of all of the components, particularly when manufactured by molding of suitable plastic or synthetic resin assures accurate dimensions and the design of all the components is such that they are readily capable of being formed by molding from plastic material.

## Claims

1. A manually operable ejector holder in combination with a cartridge (34) loaded with viscous dental filling material or cement and the cartridge having a body, having an annular collar (56) on one end and a discharge tip (64) on the front end, said holder comprising an elongate barrel (10) having forward (16) and rearward (14) ends, a plunger (18) reciprocable therein and one end (30) thereof projecting beyond said rearward end (14) of said barrel (10), a handle (22) connected to said rearward end (14) of said barrel (10) and extending substantially transversely to the axis thereof, a lever (24) manually operabe relative to said handle (22) and barrel (10) to reciprocate said plunger (18) to the front end of said barrel (10) for engagement with the cartridge (34) when disposed therein, and the forward end (16) of the barrel being cutaway longitudinally a limited distance from the forward extremity to provide a compartment (32), having sidewalls (36,38) extending over more than 180°, and the outer portions (40,42) of the sidewalls (36,38) of said compartment (32) having limited flexibility and extending toward each other a slightly lesser distance than the diameter of said compartment (32) to effect a snap-acting retaining means for a first portion of the body of the cartridge (34) when inserted into said compartment (32), an undercut groove (58) being formed in said compartment (32) within said sidewalls (36,38) to receive the annular collar (56), the sidewalls (36,38) of said compartment (32) at the rearward end thereof being recessed laterally (52,54) a greater distance than the diameter of said annular collar (56) on the cartridge to permit the collar (56) thereon to be inserted into said compartment (32) incident to being moved axially forward for disposition into said undercut groove (58), with the cartridge discharge tip (64) and a second portion of the body extending forward from the extremity of the barrel (10).

2. The combination as claimed in claim 1 wherein said undercut groove (58) at the upper ends thereof having opposite wall portions in said compartment extending toward each other a limited distance less than the diameter of the annular cartridge collar (56) to provide a seat for said collar (56) forward of the portion of said laterally recessed portion of said sidewalls (36,38) of said compartment (32) and from which said collar (56) cannot be removed laterally.

3. The combination as claimed in claim 1 or 2, wherein said lever (24) is connected to one end of said handle (22) by pivotal means (26) and extends from said pivotal means (26) past said projecting end (30) of said plunger (18), said lever (24) having an arcuate surface (70) intermediately of the ends thereof engaging said projecting end (30) of said plunger.

4. The combination as claimed in claim 1 characterised in that the sidewalls (36, 38) of said compartment (32) forwardly of said undercut groove (58) extend outwardly in a radial direction from a semicylindrical innermost surface of uniform diameter and the distance between said sidewalls (36, 38) being uniform throughout the length thereof forwardly of said undercut groove (58).

5. The combination claimed in claim 3 characterised in that said arcuate surface (31) has a centre of curvature coincident with the axis of said plunger (18) for pivotal movement of said lever (24) relative thereto when said lever (24) is moved toward said handle (22) to move said plunger (18) toward the forward end (16) of said barrel (10).

6. The combination as claimed in claim 5 for extruding viscous dental material and the like from said capsule like cartridge (34) via a discharge means (64) on one end thereof characterised in that the lever (24) is of the second class and mounted adjacent to said handle (22) and intermediately of the ends thereof, said lever having means (31) engagable with said projecting end (30) of the plunger (18) in pivotal rocking movement about the axis of said plunger (18) when said lever (24) is moved manually toward said handle (22), and the pivotal means (26) between similar adjacent ends of said handle (22) and lever (24) comprising interengaged pivot and slot means thereon operable to permit said pivotal rocking movement of said lever (24) relative to said projecting end (30) of said plunger (18).

7. The combination as claimed in claim 6 characterised in that said lever (24) and projecting end (30) of said plunger (18) have interengaging partial ball and socket means (30', 70) to effect said pivotal rocking movement therebetween, whereby manual engagement of said lever (24) and handle (22) to move the same toward each other permits a squeezing action substantially devoid of any sliding tendency in such manual contact with said lever (24).

8. The combination as claimed in claim 7 characterised in that said pivotal means on the ends of said lever and handle comprise a transverse pivot member (26) fixed to one of the same and slots receiving said pivot member (26) being formed in the other susbtantially longitudinally thereof.

9. The combination as claimed in claim 7 characterised in that the partial ball (30') is on said projecting end of the plunger (18) and coaxial therewith, and the partial socket (70) is formed in said lever (24) in a surface nearest said handle (22).

10. The combination as claimed in claim 9 characterised in that the partial ball (30') on said plunger (18) has a circumference of greater diameter than the diameter of said plunger (18), and a coiled spring (60) surrounds a portion of said plunger (18) adjacent to said partial ball (30'), the opposite ends of said spring respectively engaging said rearward end (14) of the barrel (10) and said partial ball (30').

11. The combination as claimed in claim 8 characterised in that said transverse pivot member (26) is fixed to said handle (22) and said slots being formed in the adjacent end of said lever (24), whereby said lever (24) is slidably pivotal relative to said handle (22).

12. The combination as claimed in any preceding claim wherein the front end of the body of the cartridge is closed by a hemispherical wall (62) of substantially uniform thickness, the discharge tip (64) extends from said closed end (62) of said body at an angle to the axis of said body, and there is a piston (66) having sidewalls closely complementary to the inner walls of said body and inserted into the open end thereof to form a combination closure and ejecting means for the material contained in said cartridge (34).

13. The combination according to claim 12 wherein the inner end (68) of said piston (66) is hemispherical and complementary in shape to the interior surface of the closed end of said body to effect ejection of substantially the entire contents of said cartridge (34) when said piston (66) is fully inserted into said body of the cartridge (34).

## Patentansprüche

1. Handbetätigbare Ejektorhaltereinrichtung in Kombination mit einer Patrone (34), die mit viskosem dentalen Füllungsmaterial oder Zement beladen ist und wobei die Patrone einen Körper mit einem ringförmigen Kragen (56) an einem Ende und einer Auslaßspitze (64) auf der Frontseite aufweist, wobei die Halteeinrichtrung eine längliche Führungshülse (10) mit einem vorderen Ende (16) und einem hinteren Ende (14) aufweist, einen Kolben (18), der darin verschiebbar ist und dessen eines Ende (30) über das hintere Ende (14) der Führungshülse (10) herrausragt, einen Handgriff (22), der mit dem hinteren Ende (14) der Führungshülse verbunden ist und sich im wesentlichen transversal bezüglich deren Achse erstreckt, einen Hebel (24), der relativ zu dem Handgriff (22) und der Führungshülse (10) manuell antreibbar ist, um den Kolben (18) auf das vordere Ende der Führungshülse (10) hinzubewegen, um ihn mit der darin angeordneten Patrone (34) in Eingriff zu bringen, und wobei das vordere Ende (16) der Führungshülse in Längsrichtung über eine begrenzte Strecke von dem vorderen äußersten Ende weggeschnitten ist, um eine Kammer (32) zu schaffen, mit Seitenwänden (36,38), die sich über mehr als 180° erstrecken, und die äußeren Abschnitte (40,42) der Seitenwände (36,38) der Kammer (32) eine begrenzte Flexibilität aufweisen und sich aufeinanderzu erstrecken, mit einem geringfügig kleineren Abstand als der Durchmesser der Kammer (32), so daß eine Halteeinrichtung mit Schnappwirkung für einen ersten Abschnitt des Körpers der Patrone (34) wirksam wird, wenn diese in die Kammer (32) eingesetzt ist, wobei eine Unterschnittnut (58) in der Kammer (32) mit den Seitenwänden (36,38) ausgebildet ist, zur Aufnahme des ringförmigen Kragens (56), wobei die Seitenwände (36,38) der Kammer (32) an deren hinterem Ende seitlich eine Aussparung (52,54) mit einem Abstand aufweisen, der größer ist als der Durchmesser des ringförmigen Kragens (56) der Patrone, um zu gewährleisten, daß der Kragen (56) in die Kammer (32) eingeführt werden kann, ehe er in axialer Richtung vorwärts bewegt und in die Unterschnittnut (58) eingeführt wird, wobei sich die Patronenauslaßspitze (64) und ein zweiter Abschitt des Körpers vom äußersten Ende der Führungshülse (10) nach vorwärts erstrecken.

2. Kombination gemäß Anspruch 1, wobei die Unterschnittnut (58) an deren oberen Enden gegenüberliegende Wandabschnitte in der Kammer aufweist, die sich über eine begrenzte Entfernung aufeinanderzu erstrecken, wobei die Entfernung kleiner ist als der Durchmesser des ringförmigen Kragens (56), um einen Sitz für den Kragen (56) vor dem Bereich der seitlich ausgesparten Abschnitte der Seitenwände (36,38) der Kammer (32) zu bilden, und aus dem der Kragen (56) in seitlicher Richtung nicht entnommen werden kann.

3. Kombination gemäß Anspruch 1 oder 2, wobei der Hebel (24) mit einem Ende des Handgriffs (52) durch eine Gelenkeinrichtung (26) verbunden ist und sich von der Gelenkeinrichtung (26) vorbei an dem vorspringenden Ende (30) des Kolbens (18) erstreckt, wobei der Hebel (24) zwischen seinen Enden eine bogenförmige Oberfläche (70) aufweist, mit der das vorspringende Ende (30) des Kolbens in Eingriff steht.

4. Kombination gemäß Anspruch 1, dadurch gekennzeichnet, daß sich die Seitenwände (36,38) der Kammer (321) vor der Unterschnittnut (58) von einer semizylindrischen innersten Oberfläche mit einförmigen Durchmesser in einer radialen Richtung nach außen erstrecken und der Abstand zwischen den Seitenwänden(36,38) über die gesamte Länge vor der erwähnten Unterschnittnut (58) einförmig ist.

5. Kombination gemäß Anspruch 3, dadurch gekennzeichnet, daß die bogenförmige Oberfläche (70) ein Zentrum der Krümmung aufweist, die mit der Achse des Kolbens (18) zusammenfällt, so daß die Hebelbewegung des Hebels (24) auf den Handgriff (22) den Kolben (18) auf das vordere Ende (16) der Führungshülse (10) hinbewegt.

6. Kombination gemäß Anspruch 5 zur Extrudierung von viskosem Dentalmaterial und dergleichen aus der kapselartigen Patrone (34) via eine Auslaßeinrichting (64) am einen Ende derselben, dadurch gekennzeichnet, daß der Hebel (24) zur zweiten Klasse gehört und benachbart des Handgriffs (22) befestigt ist und der Hebel zwischen seinen Enden eine Einrichtung (31) aufweist, die mit dem vorspringenden Ende (30) des Kolbens (18) drehbar angelenkt in Eingriff gebracht werden kann für eine Hin- und Herbewegung der Achse des Kolbens (18), wenn der Hebel (24) manuell auf den Handgriff (22) hinbewegt wird und wobei die Anlenkeinrichtung (26) zwischen ähnlichen benachbarten Enden des Handgriffs (22) und dem Hebel (24) eine miteinander in Eingriff stehende Zapfen- und Schlitzeinrichtung umfaßt, die in der Weise betrieben werden kann, daß eine drehbar angelenkte Hin- und Herbewegung des Hebels (24) bezüglich des vorspringenden Endes (30) des Kolbens (18) möglich ist.

7. Kombination gemäß Anspruch 6, dadurch gekennzeichnet, daß der Hebel (24) und das vorspringende Ende (30) des Kolbens (18) miteinander in Eingriff stehende teilweise Kugel- und Schaleneinrichtung (30',70) aufweisen, um die erwähnte drehbar gelagerte Hin- und Herbewegung zwischen denselben zu bewirken, wobei das manuelle Ineingriffbringen des Hebels (24) und des Handgriffs (22) unter Bewegung derselben aufeinanderzu es gestattet, eine Quetschwirkung auszuüben, die im wesentlichen frei ist von irgendeiner Abgleittendenz bei einem derartigen manuellen Kontakt mit dem Hebel (24).

8. Kombination gemäß Anspruch 7, dadurch gekennzeichnet, daß die Lagereinrichtungen am Ende des Hebels und im Handgriff ein transversales Zapfenorgan (26) umfassen, das an einem der Bauteile befestigt ist und wobei Schlitze zur Aufnahme des Zapfenorgans (26) in dem anderen der Bauteile im wesentlichen in Längsrichtung desselben ausgebildet sind.

9. Kombination gemäß Anspruch 7, dadurch gekennzeichnet, daß die Teilkugel (30') auf dem vorspringenden Ende des Kolbens (18) und koaxial mit demselben ausgebildet ist und die Teilschale (70) in dem Hebel (24) in der Oberfläche , die dem Handgriff (22) am nächsten liegt, ausgebildet ist.

10. Kombination gemäß Anspruch 9, dadurch gekennzeichnet, daß die Teilkugel (30') einen Umfangskreis mit größerem Durchmesser als der Durchmesser des Kolbens (18) aufweist und eine Federwendel (60) einen Abschnitt des Kolbens (18) benachbart der Teilkugel (30') umgibt, wobei die entgegengesetzten Enden der Feder jeweils mit dem hinteren Ende (14) der Führungshülse (10) bzw. mit der Teilkugel (30') in Eingriff stehen.

11. Kombination gemäß Anspruch 8, dadurch gekennzeichnet, daß das transversale Zapfenorgan (26) an dem Handgriff (24) fixiert ist und die Schlitze in dem benachbarten Ende des Hebels (24) ausgebildet sind, so daß der Hebel (24) bezüglich des Handgriffs (22) verschiebbar angelenkt ist.

12. Kombination gemäß einem der vorstehenden Ansprüche, wobei das vordere Ende des Körpers der Patrone durch eine halbkugelförmige Wand (62) mit im wesentlichen einförmiger Dicke verschlossen ist, die Auslaßspitze (64) sich von dem verschlossenen Ende (62) des Körpers in einem Winkel bezüglich der Achse des Körpers erstreckt und ein Kolben (66) mit Seitenwänden in enger Komplementärbeziehung zu den Innenwänden des Körpers vorgesehen ist und in das offene Ende des Körpers eingesetzt ist zur Ausbildung einer Kombination von Schließ- und Ausdrückeinrichtungen für das in der Patrone enthaltene Material.

13. Kombination gemäß Anspruch 12, wobei das innere Ende (68) des Kolbens (66) halbkugelförmig und mit einer komplementären Gestalt bezüglich der inneren Oberfläche des verschlossenen Endes des Körpers ausgebildet ist, um das Ausdrücken von im wesentlichen dem gesamten Inhalt der Patrone (34) zu bewirken, wenn der Kolben (66) vollständig in den Körper der Patrone (34) eingeschoben ist.

## Revendications

1. Porte-éjecteur actionnable manuellement en combinaison avec une cartouche (34) chargée d'un matériau visqueux de remplissage dentaire ou colle et la cartouche possédant un corps ayant un collier annulaire (56) sur une extrémité et une pointe de distribution (64) sur l'extrémité antérieure, ledit porte-éjecteur comportant un barillet allongé (10) possédant des extrémités antérieures (16) et postérieure (14), un plongeur (18) pouvant aller et venir à l'intérieur et une extrémité (30) de celui-ci faisant saillie au-delà de ladite extrémité postérieure (14) dudit barillet (10), une poignée (22) reliée à ladite extrémité postérieure (14) dudit barillet (10) et s'étendant sensiblement transversalement à l'axe de celui-ci, un levier (24) actionnable manuellement par rapport à ladite poignée (22) et audit barillet (10) pour faire aller et venir ledit plongeur (18) jusqu'à l'extrémité antérieure dudit barillet (10) pour un contact avec la cartouche (34) lorsqu'elle est disposée à l'intérieur, et l'extrémité antérieure (16) du barillet étant évidée longitudinalement sur une distance limitée à partir de l'extrémité antérieure pour constituer un compartiment (32) possédant des parois latérales (36, 38) s'étendant sur plus de 180°, et les parties extérieures (40, 42) des parois latérales (36, 38) dudit compartiment (32) possédant une flexibilité limitée et s'étendant l'une vers l'autre sur une distance légèrement moindre que le diamètre dudit compartiment (32) pour constituer un organe de retenue par encliquetage pour une première partie du corps de la cartouche (34) lorsqu'elle est introduite dans ledit compartiment (32), une gorge évidée (58) étant pratiquée dans ledit compartiment (32) à l'intérieur desdites parois latérales (36, 38) pour recevoir le collier annulaire (56), les parois latérales (36, 38) dudit compartiment (32) à l'extrémité postérieure de celui-ci étant évidées latéralement (52, 54) sur une distance supérieure au diamètre dudit collier annulaire (56) sur la cartouche pour permettre au collier (56) sur celle-ci d'être introduit dans ledit compartiment (32) déplacé axialement vers l'avant pour être disposé dans ladite gorge évidée (58), avec la pointe de distribution (64) de la cartouche et une seconde partie du corps s'étendant vers l'avant depuis l'extrémité du barillet (10).

2. Combinaison selon la revendication 1, dans laquelle ladite gorge évidée (58) a ses extrémités possédant des parties de parois opposées dans ledit compartiment s'étendant l'une vers l'autre sur une distance limitée inférieure au diamètre du collier de cartouche annulaire (56) pour constituer un siège pour ledit collier (56) en avant de la partie de ladite partie évidée latéralement desdites parties latérales (36, 38) dudit compartiment (32), et à partir duquel ledit collier (56) peut être retiré latéralement.

3. Combinaison selon la revendication 1 ou 2, dans laquelle ledit levier (24) est relié à une extrémité de ladite poignée (22) par un organe pivotant (26) et s'étend depuis ledit organe pivotant (26) devant ladite extrémité saillante (30) dudit plongeur (18), ledit levier (24) possédant une surface arquée (70) entre ses extrémités en contact de ladite extrémité saillante (30) dudit plongeur.

4. Combinaison selon la revendication 1, caractérisée en ce que les parois latérales (36, 38) dudit compartiment (32) en avant de ladite gorge évidée (58) s'étendent vers l'extérieur dans une direction radiale depuis une surface interne semi-cylindrique de diamètre uniforme et la distance entre lesdites parois latérales (36, 38) étant uniforme sur toute leur longueur en avant de ladite gorge évidée (58).

5. Combinaison selon la revendication 3, caractérisée en ce que ladite surface arquée (31) possède un centre de courbure coïncidant avec l'axe dudit plongeur (18) en vue d'un mouvement de pivotement dudit levier (24) par rapport à celui-ci lorsque ledit levier (24) est déplacé vers ladite poignée (22) pour déplacer ledit plongeur (18) vers l'extrémité antérieure (16) dudit barillet (10).

6. Combinaison selon la revendication 5, en vue d'extruder un matériau dentaire visqueux et analogues à partir de ladite cartouche (34) analogue à une capsule par l'intermédiaire d'un organe de distribution (64) sur une de ses extrémités, caractérisée en ce que le levier (24) est du second ordre et monté adjacent à ladite poignée (22) et entre les extrémités de celle-ci, ledit levier possédant des moyens (31) pouvant venir en contact de ladite extrémité saillante (30) du plongeur (18) au cours d'un mouvement de basculement pivotant autour de l'axe dudit plongeur (18) lorsque ledit levier (24) est déplacé manuellement vers ladite poignée (22), et les organes de pivotement (36) entre des extrémités adjacentes similaires de ladite poignée (22) et du levier (24) comportant des moyens de pivotement et de fente en contact les uns des autres sur celui-ci, actionnables pour permettre ledit mouvement de basculement pivotant dudit levier (24) par rapport à ladite extrémité saillante (30) dudit plongeur (18).

7. Combinaison selon la revendication 6, caractérisée en ce que ledit levier (24) et l'extrémité saillante (30) dudit plongeur (18) possèdent des joints à rotule partiels s'interpénétrant (30', 70) pour effectuer ledit mouvement de basculement pivotant entre eux, de telle sorte qu'un contact manuel dudit levier (24) et de la poignée (22) pour les déplacer l'un vers l'autre permet une action de compression sensiblement exempte d'une tendance quelconque au glissement dans un tel contact manuel avec ledit levier (24).

8. Combinaison selon la revendication 7, caractérisée en ce que lesdits moyens pivotants sur les extrémités dudit levier et de la poignée comportent un pivot transversal (26) fixé à l'un d'eux et des fentes recevant ledit pivot (26) étant pratiquées dans l'autre sensiblement longitudinalement à celui-ci.

9. Combinaison selon la revendication 7, caractérisée en ce que la bille partielle (30') se trouve sur ladite extrémité saillante du plongeur (18) et coaxiale avec lui, et la douille partielle (70) est réalisée dans ledit levier (24) dans une surface la plus proche de ladite poignée (22).

10. Combinaison selon la revendication 9, caractérisée en ce que la bille partielle (30') sur ledit plongeur (18) possède une circonférence de diamètre supérieure au diamètre dudit plongeur (18), et un ressort hélicoïdal (60) entoure une partie dudit plongeur (18) adjacente à ladite bille partielle (30'), les extrémités opposées dudit ressort étant en contact respectivement de ladite extrémité postérieure (14) du barillet (10) et ladite bille partielle (30').

11. Combinaison selon la revendication 8, caractérisée en ce que ledit pivot transversal (26) est fixé à ladite poignée (22) et lesdites fentes étant réalisées dans l'extrémité adjacente dudit levier (24), de telle sorte que ledit levier (24) puisse pivoter à coulissement par rapport à ladite poignée (22).

12. Combinaison selon l'une quelconque des revendications précédentes, dans laquelle l'extrémité antérieure du corps de la cartouche est fermée par une paroi hémisphérique (62) d'épaisseur sensiblement uniforme, la pointe de distribution (64) s'étend depuis ladite extrémité fermée (62) dudit corps selon un angle par rapport à l'axe dudit corps, et un piston (66) étant prévu possédant des parois latérales étroitement complémentaires aux parois internes dudit corps et introduites dans l'extrémité ouverte de celui-ci pour former une enceinte de la combinaison et des moyens d'éjection pour le matériau contenu dans ladite cartouche (34).

13. Combinaison selon la revendication 12, dans laquelle l'extrémité interne (68) dudit piston (66) est hémisphérique et de forme complémentaire à la surface interne de l'extrémité fermée dudit corps pour effectuer l'éjection de pratiquement la totalité du contenu de ladite cartouche (34) lorsque ledit piston (66) est complètement introduit dans ledit corps de la cartouche (34).
